# EUROPÄISCHE PATENTANMELDUNG

(11) **EP 1 914 546 A2**
(43) Veröffentlichungstag der Anmeldung: **23.04.2008**
(21) Anmeldenummer: 07020118.1
(22) Anmeldetag: 15.10.2007
(51) Int. Cl.: G01N 33/28

(54) **Vorrichtung und Verfahren zur Bestimmung der Gebrauchseigenschaften eines Schmiermittels**

(30) Priorität: 19.10.2006 DE 102006049349
(71) Anmelder: IAV GmbH Ingenieurgesellschaft Auto und Verkehr, 10587 Berlin (DE)
(72) Erfinder: Berger, Ulrich, Dr.-Ing., 38518 Gifhorn (DE); Lappe, Stefan, 38553 Wasbüttel (DE); Wilkens, Olaf, 38108 Braunschweig (DE)
(74) Vertreter: Buss, Fritz Gerd

(57) **Zusammenfassung**

Die Erfindung betrifft eine Vorrichtung und ein Verfahren zur Analyse eines Gases hinsichtlich seines Kohlenwasserstoffgehaltes, vorzugsweise zur Anwendung auf die Bestimmung des Kraftstoffeintrags in ein Motorenöl. In einer dichtend abschließbaren Probenkammer (1) wird eine Schmiermittelprobe (2) aufgeheizt und mittels eines Trägergases werden die flüchtigen Bestandteile aus der Schmiermittelprobe (2) ausgetragen. Das Trägergas wird nachfolgend über einen Oxidationskatalysator (14) geleitet und oxidiert, wobei das Reaktionsprodukt der Oxidation wenigstens einem Gassensor (12) zugeführt wird, der die Konzentration des im Trägergas enthaltenen CO₂ oder zusätzlich O₂ ermittelt.

## Beschreibung

### Technisches Gebiet

Die Erfindung betrifft eine Vorrichtung zur Bestimmung der Gebrauchseigenschaften eines Schmiermittels, bei welcher zur Bestimmung der Gebrauchseigenschaften des Schmiermittels eine Analyse von Gasen hinsichtlich deren Anteils an Kohlenwasserstoffen erfolgt nach dem Oberbegriff des Anspruches 1 und ein Verfahren gemäß dem Oberbegriff des Anspruches 5.
Eine schnelle unkomplizierte Analyse eines Gases hinsichtlich seines Anteils an Kohlenwasserstoffen ist für Aerosole hochsiedender Kohlenwasserstoffe in Gasen, z. B. Druckluft, die ölfrei sein soll oder die Emissionsüberwachungen, z. B. an Tanklagern o. ä. notwendig. Eine bevorzugte Anwendung stellt weiterhin die Analyse von Motorenölen dar. Die Gebrauchseigenschaften eines Motorenöls sind insbesondere hinsichtlich der Schmierwirkung für die Lebensdauer eines Motors eine entscheidende Größe. Sie bestimmen die notwendigen Wechselintervalle und sind damit sowohl für das mechanische Zusammenspiel der Bauteile zu betrachten, als auch hinsichtlich der Erneuerung des Schmiermittels eine wirtschaftliche Komponente. Insbesondere der Kraftstoffeintrag in das für Motoren als Schmiermittel verwendete Motorenöl wirkt sich negativ auf die Gebrauchseigenschaften aus, indem die Schmierwirkung herabgesetzt wird. Es ist daher von Bedeutung, die im Schmiermittel vorhandenen Bestandteile analysieren zu können und insbesondere für den im Fahrzeug wichtigsten Fall, den Eintrag von Kraftstoff in das Schmiermittel, eine Analyse des Schmiermittels vorzunehmen. Eine solche Analyse ist beispielsweise beim Betreiben eines Motors unter Testbedingungen, beispielsweise bei Dauerlaufversuchen auf einem Prüfstand, notwendig. Die Analyse muss dabei möglichst schnell und mit wenig Aufwand am Prüfstand realisierbar sein.

Vorbekannt ist aus der DE 198 49 195 C1 eine Vorrichtung und ein Verfahren zur Messung des Kohlenwasserstoffgehaltes in einem Messgas. Hierfür werden sogenannte Flammionisationsdetektoren FID eingesetzt, welche summarisch den Gesamtgehalt an Kohlenwasserstoffen im Messgas bestimmen. Die Verwendung der FID ist nachteilig, da beispielsweise bei der Analyse einer Schmiermittelprobe der Öldampf kondensiert, sobald er die Verdampfungskammer verlässt. Er würde sich in Zuleitungen und/oder dem FID ablagern und dort die Strömung beeinflussen sowie falsche Messungen bewirken. Das beschriebene Verfahren nutzt zwei FID, um mit einem die Gesamtkonzentration an Kohlenwasserstoffen im Messgas zu bestimmen. Der Strom des Messgases wird dabei geteilt, um einen Teil über einen in einem Bypass befindlichen Oxidationskatalysator zu leiten und die Kohlenwasserstoffe zu Kohlendioxyd und Wasser zu oxidieren, wobei in diesem Teilstrom lediglich das im Messgas enthaltene Methan aus der Gruppe der Kohlenwasserstoffe verbleibt. Nachfolgend wird mit dem zweiten FID eine Analyse des Gasstromes, der über den Oxidationskatalysator geleitet wird, durchgeführt. Der zweite FID bestimmt dabei lediglich die Methankonzentration, so dass der Methananteil nachfolgend von der Gesamtsumme abgezogen werden kann.

Vorbekannt ist aus der DE 103 27 625 A1 eine Vorrichtung, bei welcher ein zu prüfendes Schmiermittel in eine Probenkammer eingebracht wird. Die Probenkammer kann dabei Mittel enthalten, welche vorbestimmte physikalische Randbedingungen, wie Druck und Temperatur in der Probenkammer einstellen. Die Probenkammer wird zusätzlich mit einem Trägergas gefüllt, welches sich mit den flüchtigen Bestandteilen der Probe anreichert. Das Trägergas wird nachfolgend einer Sensorkammer zugeführt, in welcher die Bestandteile mittels wenigstens eines Gassensors analysiert werden.
Ein Nachteil des Verfahrens ist die große Anzahl verschiedener Kohlenwasserstoffe, welche gleichzeitig aus der Probe ausdampfen. Eine schnelle synchrone Analyse aller Kohlenwasserstoffe ist aufwändig. Für die schnelle Messung einer Probe zur Beurteilung des Kraftstoffeintrages in das Schmiermittel eines Motors ist das Verfahren daher ungeeignet.

Der Erfindung liegt die Aufgabe zugrunde, eine einfache Vorrichtung und ein Verfahren zur Bestimmung der Gebrauchseigenschaften eines Schmiermittels bei welcher zur Bestimmung der Gebrauchseigenschaften des Schmiermittels eine Analyse von Gasen hinsichtlich deren Anteils an Kohlenwasserstoffen erfolgt zu schaffen, welches mit einem geringen Messaufwand auskommt. Weiterhin wird eine auf der Gasanalyse beruhende Vorrichtung und Verfahren zur Ermittlung der Gebrauchseigenschaften eines Schmiermittels angegeben, welches für eine schnelle Analyse des Kraftstoffeintrages in das Schmiermittel eines Motors geeignet ist.

Die Aufgabe wird durch ein Verfahren und eine Vorrichtung gemäß der unabhängigen Ansprüche 1 und 5 gelöst. Vorteilhafte Ausgestaltungen sind den Unteransprüchen zu entnehmen.

Erfindungsgemäß vorteilhaft wird das zu analysierende Gas über einen beheizbaren Oxidationskatalysator geleitet, der bei Betriebstemperatur die im Gas enthaltenen Kohlenwasserstoffe an dessen katalytisch wirksamer Oberfläche zu Kohlendioxid und Wasser umwandelt. Nachfolgend erfolgt eine Messung der Kohlendioxidkonzentration, welche als Reaktionsprodukt der Oxidation der Kohlenwasserstoffe verbleibt.

Erfindungsgemäß vorteilhaft wird die Vorrichtung zur Analyse von Gasen für die Bestimmung der Gebrauchseigenschaften eines Schmiermittels genutzt. In einer dichtend abschließbaren Probenkammer befindet sich eine Schmiermittelprobe, welche über eine Heizung sowie eine Zu- sowie Abführung für ein Trägergas in die Probenkammer verfügt. Das Trägergas wird nachfolgend über einen Oxidationskatalysator geleitet, an welchem die im Trägergas befindlichen flüchtigen Anteile der Schmiermittelprobe oxidiert werden. Das Oxidationsprodukt wird dann wenigstens einem Gassensor zugeführt. In vorteilhafter Weise erfolgt hier keine Analyse von einzelnen Kohlenwasserstoffen aus dem Trägergas, sondern eine Summenbetrachtung über alle verflüchtigten Bestandteile der Schmiermittelprobe. Die Oxidation der aus der Schmiermittelprobe ausgetragenen flüchtigen Bestandteile an der katalytisch wirksamen Oberfläche erzeugt CO₂ und H₂O. Das Gasgemisch wird hinsichtlich seiner CO₂- und/der O₂-Konzentration analysiert. Durch die Analyse des Oxidationsproduktes wird somit ein leicht analysierbares Gasgemisch erzeugt, wobei aus der Messung lediglich der CO₂- und/der O₂-Konzentration Rückschlüsse auf den Kraftstoffeintrag erfolgen.

Erfindungsgemäß vorteilhaft weist die Probenkammer eine Heizung auf, um die enthaltene Schmiermittelprobe aufzuheizen. Weiterhin ist der Oxidationskatalysator zum Erreichen seiner Betriebstemperatur beheizbar. Erfindungsgemäß vorteilhaft befinden sich Probenkammer und Oxidationskatalysator in einem gemeinsam beheizbaren Raum, so dass die Oxidation und der Austrag der flüchtigen Bestandteile aus der Schmiermittelprobe in einem gemeinsamen beheizten Raum erfolgen, was die problematischen Kondensationserscheinungen der Ölproben, beispielsweise in den Zuleitungen zu dem/n Gassensor/en vermeidet.
In einer Weiterbildung der Erfindung wird dem Oxidationskatalysator für die Umwandlung der aus der Schmiermittelprobe verflüchtigten Bestandteile Luft oder Sauerstoff zugeführt. Dies ist insbesondere erforderlich, wenn als Trägergas ein Inertgas verwendet wird, um die vollständige Umwandlung der aus der Schmiermittelprobe verflüchtigten Bestandteile zu erreichen.

Erfindungsgemäß vorteilhaft kann als Gassensor ein CO₂-Messgerät verwendet werden, welches lediglich auf die Erfassung einer Gaskomponente ausgelegt ist. Weiterhin kann ein O₂-Sensor verwendet werden, um den Restsauerstoff zu analysieren. Weiterhin kann parallel eine Messung der Konzentration von CO₂ und O₂ erfolgen, wobei kombinierte CO₂- und O₂-Messgeräte am Markt erhältlich sind. In einer vorteilhaften Ausführung ist der Gassensor ein Massenspektrometer, welches CO₂ und O₂ messen kann.
Parallel zur Messung der Konzentration von CO₂ und/oder Sauerstoff wird die Temperatur der Schmiermittelprobe gemessen. Damit wird eine temperaturaufgelöste Messung der Konzentration ermöglicht. Die Konzentration, beispielsweise von CO₂ kann somit einer bestimmten Probentemperatur zugeordnet werden. Die niedrig siedenden Kohlenwasserstoffe, welche z. B. aus dem Eintrag von Otto-Kraftstoff herrühren, verdampfen im Wesentlichen bei Temperaturen zwischen 150 und 250°C und bilden sich als messbare Konzentrationserhöhung von CO₂ im Trägergas ab.

Erfindungsgemäß erfolgt durch die Messung der Sauerstoffkonzentration eine Verfolgung des Sauerstoffverlustes bei der Oxidation, woraus das H/C-Verhältnis in der Probe bestimmt wird. Hieraus können Rückschlüsse über die im Gas bzw. im Schmiermittel enthaltenen Bestandteile gezogen werden. Für die Messung des Sauerstoffverlustes muss die dem Oxidationskatalysator zugeführte Sauerstoffmenge ermittelt werden.

Nachfolgend wird die Erfindung anhand eines bevorzugten Ausführungsbeispiels beschrieben.

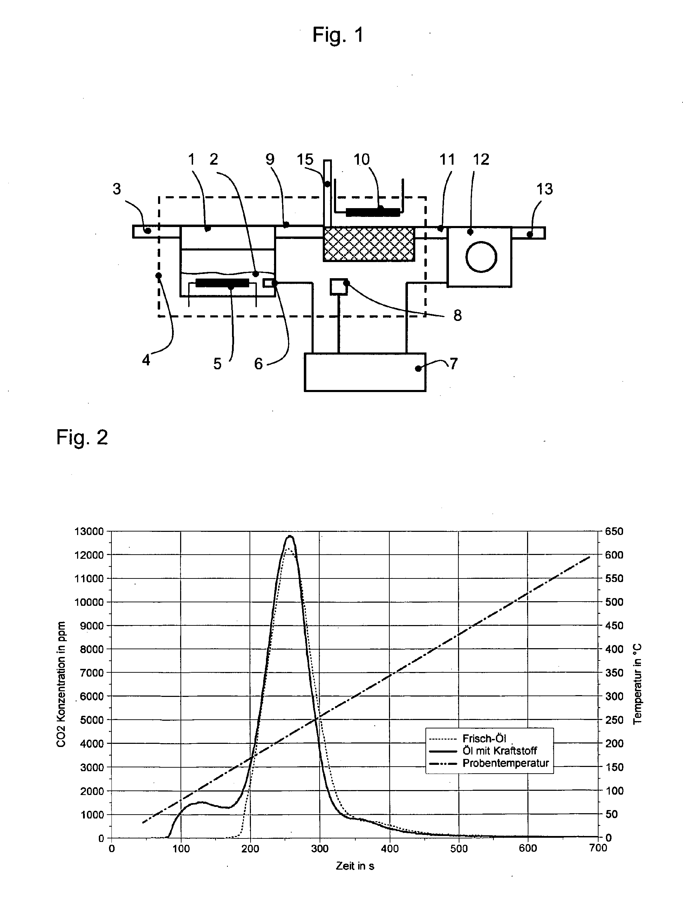

Es zeigt Figur 1 eine Prinzipdarstellung der erfindungsgemäßen Vorrichtung. Innerhalb einer Probenkammer 1 befindet sich eine zu analysierende Schmiermittelprobe 2. Die jeweils zu analysierende Schmiermittelprobe 2 wird dabei manuell in die Probenkammer 1 eingebracht, wobei eine automatische Zuführung 3 aus dem Schmierkreislauf eines Motors vorgesehen sein kann. Die Schmiermittelprobe 2 muss zur Analyse gasdicht gegenüber der Umgebung verschlossen werden, wobei ein Trägergas (vorzugsweise ein Inertgas) über die Zuführung 3 in die Probenkammer 1 eingeleitet wird. Das Trägergas wird dabei kontinuierlich über oder durch die Schmiermittelprobe 2 geleitet. In einer weiteren Variante kann das Inertgas portionsweise in die Probenkammer 1 geleitet werden und dort für einen Zeitraum abgeschlossen verbleiben. Über eine Heizung 5 erfolgt ein Aufheizen der Schmiermittelprobe 2. Ein Temperatursensor A 6 in der Probenkammer 1 misst dabei den Temperaturverlauf in der Schmiermittelprobe 2. In einer Variante der Erfindung wird dabei eine kleine Menge einer Schmiermittelprobe 2 mittels einer rampenförmigen Temperaturerhöhung komplett verdampft und über das Trägergas kontinuierlich ausgetragen. Eine weitere alternative Variante ist die stufenweise Aufheizung, wobei die Schmiermittelprobe 2 über einen Zeitraum auf einer konstanten Temperatur verbleibt. Das über dem Flüssigkeitsspiegel der Schmiermittelprobe 2 befindliche Inertgas reichert sich mit den flüchtigen Bestandteilen aus der Schmiermittelprobe 2 an. Es kann ebenfalls in einer weiteren Ausführung durch die Probe geleitet werden. Über die Zuführung 3 wird bei einem stufenweisen Austragen weiteres Trägergas in die Probenkammer 1 eingeleitet, welches sich bei der nachfolgenden weiteren Aufheizung mit den flüchtigen Bestandteilen der Schmiermittelprobe 2 anreichert. Für jede der beschriebenen Varianten ist die nachfolgend beschriebene Weiterbehandlung des mit den flüchtigen Bestandteilen der Schmiermittelprobe 2 angereicherten Trägergases gleich.

Das Trägergas wird nachfolgend über die Abführung 9 einem Oxidationskatalysator 14 zugeführt. An der katalytisch wirksamen Oberfläche des Oxidationskatalysators 14 werden die flüchtigen Bestandteile der Schmiermittelprobe 2 oxidiert. Hierfür wird dem Oxidationskatalysator 14 Luft oder Sauerstoff, der für die Oxidation der flüchtigen Bestandteile aus der Schmiermittelprobe 2 erforderlich ist, über eine Leitung 15 zugeführt. Der Oxidationskatalysator 14 wird mit einer Heizung 10 auf Betriebstemperatur gebracht bzw. gehalten (minimal 400°C). Der Oxidationskatalysator 14 ist dabei in einer vorteilhaften Ausführung ein herkömmlicher Fahrzeug-Oxidationskatalysator, der beispielsweise auf einem Träger aus Aluminiumoxid eine katalytisch aktive Schicht aus vorzugsweise Platin aufweist. Die Heizung 10 des Oxidationskatalysators 14 kann gleichzeitig als Heizung 10 für die innerhalb des Gehäuses 4 angeordneten Teile und Leitungen dienen, um in diesen einen Niederschlag der schwersiedenden Bestandteile aus der Schmiermittelprobe 2 zu verhindern.
In einer Ausführung der Vorrichtung ist die Probenkammer 1 außerhalb des beheizten Raumes, in dem sich der Oxidationskatalysator 14 befindet, angeordnet. Es wird dabei eine Schmiermitteltemperatur von bis zu 90°C eingestellt. Hierbei verdampfen die höher siedenden Komponenten nicht sofort vollständig, sondern sind entsprechend ihrer Dampfdrücke im Trägergas enthalten. Dadurch werden sie allmählich ausgetragen und geben je nach Menge lange Plateaus oder auch Peaks bei der nachfolgenden Analyse des Gases. Diese Methode muss anhand einer Frischölprobe kalibriert werden. In einer alternativen Variante wird eine kleine Menge einer Ölprobe komplett verdampft. Hier wird in einer Art Differential-Thermoanalyse der Kraftstoff über die mathematische Analyse des CO₂-T-Verlaufes bestimmt. Die Probenkammer 1 wird nach dem Probeneinbau in den Ofen eingebracht, wo die rasche Aufheizung und temperaturabhängige Verdampfung erfolgt. Nach der Oxidation der im Trägergas enthaltenen, aus der Schmiermittelprobe 2 ausgetragenen Kohlenwasserstoffe an der Oxidationskatalysatoroberfläche wird der gasförmige Anteil des Reaktionsproduktes, das aus CO₂ und verbliebenem Sauerstoff sowie dem Trägergas besteht, über eine Zuleitung 11 einem Gassensor 12 zugeführt und kann diesen über eine Ableitung 13 verlassen. Der Gassensor 12 wertet das Gasgemisch hinsichtlich der Konzentration des enthaltenen Kohlendioxides aus. Es wird dabei die Gesamtkonzentration der zu Kohlendioxid und Wasser oxidierten flüchtigen Bestandteile der Schmiermittelprobe 2 gemessen. Um eine Zuordnung der Konzentration zu einzelnen Bestandteilen zu erhalten, wird parallel zur Konzentrationsmessung mit dem Gassensor 12 die Temperatur T in der Gassensor 12 die Temperatur T in der Probenkammer 1 mittels des Temperatursensors A 6 gemessen. Der Kraftstoffeintrag kann durch das unterschiedliche Siedeverhalten von Kraftstoff und Schmiermittel bestimmt werden. Zur Auswertung der Gaskonzentration liegen einer Auswerteeinheit 7 die Signale des Temperatursensors A 6 und des Gassensors 12 über Datenleitungen an. Weiterhin kann ein Temperatursensor B 8 die Temperatur T innerhalb des Gehäuses 4 ermitteln. Diese Temperaturmessung dient der Überwachung der Temperatur im Gehäuse 4, um einen Niederschlag der schwer siedenden Bestandteile in den Leitungen zu verhindern, wobei weiterhin die Oxidationskatalysatortemperatur überwacht werden kann. Der Eintrag von Inertgas in die Probenkammer 1 sowie von Sauerstoff oder Luft zum Oxidationskatalysator 14 ist über nicht gezeigte Ventile steuerbar. Der Eintrag von Sauerstoff oder Luft erfolgt dabei über eine Leitung 15 zum Oxidationskatalysator 14. Weiterhin kann eine Messung der eingebrachten Volumina vorgesehen sein. Sowohl die Trägergasmenge als auch die dem Oxidationskatalysator 14 zugeführte Sauerstoff- oder Luftmenge kann mit einer geeigneten Messtechnik, bspw. einem Gasdurchflussmessgerät, ermittelt werden.

Figur 2 zeigt in einem Diagramm eine qualitative Betrachtung der vom Gassensor 12 gemessenen Konzentration von CO₂ über der in der Probenkammer 1 eingestellten Temperatur T. Es handelt sich dabei um einen typischen Verlauf der Verdampfung einer kleinen Menge einer Schmiermittelprobe 2. Die strichliert dargestellte Kurve zeigt die Messwerte für eine Probe eines Frischöls. Es erfolgt eine Messung der Konzentration von CO₂, welches durch die Oxidation der in dem Trägergas vorhandenen Kohlenwasserstoffe, die als flüchtige Bestandteile aus der Schmiermittelprobe 2 entstammen, entsteht. Aufgrund der Siedeeigenschaften von Motorenölen beginnt ein Austrag von Kohlenwasserstoffen in das Trägergas bei einer Temperatur von 190°C bis 240°C. Das Austragen des Öls erfolgt bis zu einer Temperatur von 360°C (max. 400°C). Bei dieser Temperatur ist die Schmiermittelprobe 2 weitestgehend verdampft, so dass nachfolgend keine Kohlenwasserstoffe mehr an dem Oxidationskatalysator 14 oxidiert werden, und bei Temperaturwerten > 400°C kein CO₂ als Oxidationsprodukt mehr mittels des Gassensors 12 messbar ist. Die aus der Frischölprobe stammenden Kohlenwasserstoffe bilden sich über den gesamten Temperaturbereich zwischen 190°C und 360°C in der Konzentration des Kohlendioxids ab. Im Unterschied dazu zeigt die mit einer durchgezogenen Linie gekennzeichnete Kurve in dem Diagramm eine Überhöhung der Konzentration bei einer Temperatur der Schmiermittelprobe 2 zwischen 90°C und 180°C. Es handelt sich bei der Schmierstoffprobe um ein mit Kraftstoff versetztes Motorenöl. Die Überhöhung der Konzentration resultiert aus den im Vergleich zum Motorenöl leicht siedenden Kohlenwasserstoffen. Aufgrund der Konzentration von Kohlendioxid im Trägergas, welches ein Oxidationsprodukt der aus der Schmierölprobe bei einer Temperatur zwischen 90°C und 180°C ausgetragenen Kohlenwasserstoffe ist, kann auf den Anteil des in der Schmiermittelprobe 2 enthaltenen Kraftstoffs geschlossen werden. Die Überhöhung kann beispielsweise mittels der Integration der Messkurven beurteilt werden.

Eine weitere Möglichkeit bietet die Analyse des Gasgemisches hinsichtlich des enthaltenen Sauerstoffes. Über die Analyse des Sauerstoffverlustes bei der Oxidation ist eine Beurteilung des H/C-Verhältnisses der bei der jeweiligen Temperatur ausgetragenen Kohlenwasserstoffe möglich. Hierfür muss der dem Oxidationskatalysator zugeführte Sauerstoffanteil gemessen werden. Auf Basis der Messung des im Inertgas vorhandenen Kohlendioxides und des restlichen Sauerstoffanteils kann das H/C-Verhältnis der Schmierölprobe aus dem bei der Oxidation verbrauchten Sauerstoff bestimmt werden. Im Oxidationskatalysator werden aus je 2 H-Atomen und einem 0-Atom H₂O-Moleküle erzeugt, sowie aus einem C und einem O₂-Molekül je ein CO₂ Molekül. Die CO₂-Kurve ist daher ein Maß, wie viel O₂ für die Oxidation des Kohlenstoffes verbraucht wurde. Misst man zusätzlich O₂, dann wurde alles was mehr verbraucht wurde, als durch CO₂-Bildung begründet, vom Wasserstoff zur H₂O-Bildung verbraucht. Daraus wird summarisch das H/C-Verhältnis aller verdampften Kohlenwasserstoffe berechnet.

### Bezugszeichenliste

- 1: Probenkammer
- 2: Schmiermittelprobe
- 3: Zuführung
- 4: Gehäuse
- 5: Heizung Probenkammer
- 6: Temperatursensor A (Probenkammer)
- 7: Auswerteeinheit
- 8: Temperatursensor B
- 9: Abführung
- 10: Heizung (Gehäuse)
- 11: Zuleitung (Gassensor)
- 12: Gassensor
- 13: Ableitung
- 14: Oxidationskatalysator
- 15: Leitung
- T: Temperatur

## Patentansprüche

1. Vorrichtung zur Bestimmung der Gebrauchseigenschaften eines Schmiermittels, bei welcher zur Bestimmung der Gebrauchseigenschaften des Schmiermittels eine Analyse von Gasen hinsichtlich deren Anteils an Kohlenwasserstoffen erfolgt, bei denen das Gas einem beheizbaren Oxidationskatalysator (14) über eine Abführung (9) zugeführt wird, wobei der Oxidationskatalysator (14) eine Zuleitung (11) aufweist und stromabwärts des Oxidationskatalysators (14) Gassensoren angeordnet sind, und ein Gassensor (12) ein die Konzentration des im Gas enthaltenen, CO₂ messender Sensor ist und der Oxidationskatalysator (14) eine katalytisch wirksame Beschichtung aufweist, welche die Oxidation der enthaltenen Kohlenwasserstoffe zu CO₂ und H₂O begünstigt,
wobei wenigstens ein weiterer Gassensor vorhanden ist, mittels dem zusätzlich die O₂-Konzentration messbar ist, wobei dem Oxidationskatalysator (14) Sauerstoff zugeführt wird und die zugeführte Menge an Sauerstoff gemessen wird,
und dass den Gassensoren (12) ein Gas zugeführt wird, welches aus einem Trägergas und einem Messgas besteht, wobei das Trägergas einer dichtend abschließbaren Probenkammer (1), in welche eine Schmiermittelprobe (2) eingebracht ist, entnehmbar ist, wobei die Probenkammer (1) beheizbar ist und eine Einrichtung zur Messung der Temperatur (T) der Schmiermittelprobe (2) und eine Zu- (3) sowie Abführung (9) für das Trägergas aufweist, und das aus der Probenkammer (1) entstammende Gasgemisch aus Mess- und Trägergas über die Abführung (9) dem Oxidationskatalysator (14) zuführbar ist und stromabwärts des Oxidationskatalysators (14) der Gassensor (12) zur Messung des CO₂-Gehaltes sowie der weitere Gassensor, der zur Messung der O₂-Konzentration angeordnet sind, und
einer Auswerteeinheit (7) das Signal des Temperatursensors A (6) und das Signal des/der Gassensoren (12) und das Signal eines Messgerätes für den dem Oxidationskatalysator (14) zugeführten Sauerstoffanteil und das Signal eines Messgerätes für das der Probenkammer (1) zugeführte Trägergas einer Auswerteeinheit (7) anliegt.

2. Vorrichtung nach einem der vorherigen Ansprüche,
**dadurch gekennzeichnet, dass**
der Oxidationskatalysator (14) selbst oder dessen Umgebung beheizbar ist und über eine weitere Leitung (15) dem Oxidationskatalysator (14) Sauerstoff zuführbar ist.

3. Vorrichtung nach einem der vorherigen Ansprüche,
**dadurch gekennzeichnet, dass**
zur Messung des Volumens des über die Leitung (15) dem Oxidationskatalysator (14) zugeführten Sauerstoffs und des der Probenkammer (1) zugeführten Trägergases jeweils einem Messgerät angeordnet ist.

4. Vorrichtung nach einem der vorhergehenden Ansprüche,
**dadurch gekennzeichnet, dass**
die Probenkammer (1) und der Oxidationskatalysator (14) in einem gemeinsam beheizbaren Gehäuse (4) angeordnet sind.

5. Verfahren zur Bestimmung der Gebrauchseigenschaften eines Schmiermittels, bei welchem zur Bestimmung der Gebrauchseigenschaften des Schmiermittels eine Analyse des Kohlenwasserstoffanteils eines Gases erfolgt, welches einem Oxidationskatalysator zugeführt und aus der gemessenen CO₂-Konzentration der Anteil an Kohlenwasserstoffen ermittelt wird,
wobei der Gassensor ein Gasgemisch aus einem Messgas und einem Trägergas auswertet und das Trägergas in eine Probenkammer (1) geleitet wird, in die eine Schmiermittelprobe (2) eingebracht wurde, wobei die Probenkammer (1) beheizt wird und über eine Zu- (3) bzw. Abführung (9) des Trägergases die flüchtigen Bestandteile der Schmiermittelprobe (2) aus der Probenkammer (1) ausgetragen werden, wobei die Temperatur (T) der Schmiermittelprobe (2) gemessen wird, das Trägergas mit den in diesem befindlichen flüchtigen Bestandteilen der Schmiermittelprobe (2) dem Oxidationskatalysator (14) zugeführt und unter Zugabe von Luft oder Sauerstoff oxidiert wird, wobei das Gasgemisch nach der Oxidation hinsichtlich seiner CO₂-Konzentration von wenigstens einem Gassensor (12) gemessen wird und
parallel zur Messung von CO₂ die Temperatur (T) in der Probenkammer (1) gemessen und aus der temperaturaufgelösten Messung der Konzentration von CO₂ der Kraftstoffeintrag in die Probe ermittelt werden, indem die CO₂-Konzentration in einem Temperaturbereich der Schmiermittelprobe (2) zwischen 90°C und 220°C, bevorzugt zwischen 100°C und 180°C, betrachtet wird, wobei durch einen Vergleich der Messung mit einer vorher für verschiedene Kraftstoffeinträge ermittelten CO₂-Konzentration auf den Kraftstoffeintrag geschlossen wird.

6. Verfahren nach Anspruch 5,
**dadurch gekennzeichnet, dass**
zusätzlich die O₂-Konzentration gemessen und ausgewertet wird.

7. Verfahren nach Anspruch 6,
**dadurch gekennzeichnet, dass**
dem Oxidationskatalysator (14) Sauerstoff zugeführt wird und die Menge des zugeführten Sauerstoffes gemessen wird, wobei das Gas nach dem Oxidationskatalysator (14) weiterhin hinsichtlich der O₂-Konzentration ausgewertet wird und damit aus dem Sauerstoffverlust das H/C-Verhältnis bestimmt wird.

8. Verfahren nach einem der vorhergehenden Ansprüche 6-7,
**dadurch gekennzeichnet, dass**
parallel während der Messung der Sauerstoffkonzentration eine Auswertung des Sauerstoffverlustes bei der Oxidation auf Basis der Differenz des dem Oxidationskatalysator (14) zugeführten Anteils an Sauerstoff und dem vom Gassensor (12) nach der Oxidation gemessenen Anteils an restlichem Sauerstoff erfolgt und daraus das H/C-Verhältnis in der Schmiermittelprobe (2) bestimmt wird.
